# EUROPÄISCHE PATENTSCHRIFT

(11) **EP 1 654 996 B1**
(45) Veröffentlichungstag und Bekanntmachung des Hinweises auf die Patenterteilung: **31.01.2007**
(21) Anmeldenummer: 06001021.2
(22) Anmeldetag: 07.06.2002
(51) Int. Cl.: A61B 17/88

(54) **Befestigungswerkzeug für eine Knochenschraube**
Tool for securing a bone screw
Outil de fixation pour une vis d'osteosynthèse

(30) Priorität: 27.07.2001 DE 10136129
(43) Veröffentlichungstag der Anmeldung: 10.05.2006
(62) Teilanmeldung aus: 02012710.6
(73) Patentinhaber: BIEDERMANN MOTECH GmbH, 78054 VS-Schwenningen (DE)
(72) Erfinder: Biedermann, Lutz, 78048 VS-Villingen (DE); Harms, Jürgen, 76227 Karlsruhe (DE)
(74) Vertreter: Hofer, Dorothea

(56) Entgegenhaltungen:
- US-A- 5 716 356
- US-B1- 6 251 112
- US-B1- 6 258 090

## Beschreibung

Die Erfindung betrifft ein Befestigungswerkzeug zum Fixieren einer Knochenschraube.

Eine Knochenschraube ist beispielsweise aus der EP 0 483 242 B1 bekannt. Des weiteren ist eine Knochenschraube gemäß dem Oberbegriff des Patentanspruchs 1 aus der EP 0 614 649 A1 bekannt. Bei diesen Knochenschrauben werden hohe Drehmomente beim Festziehen der Schrauben sowohl zum Fixieren des Schraubenelementes als auch zum Fixieren des Stabes aufgebracht. Da die Schrauben während der Operation - nach Einlegen des Stabes - vom Operateur endfest angezogen werden müssen, besteht dabei die Gefahr, den entsprechenden Wirbel zu verletzen. Werden die Schrauben nicht ausreichend endfest angezogen, besteht die Gefahr, daß sich die Schrauben/Stabverbindungen lösen und die Knochenschraube ihre fixierte Position verliert.

Aus der US-B1-6258090 ist ein Set bestehend aus einem Befestigungswerkzeug zum Fixieren eines Verschlusselements einer Knochenschraube, einem Werkzeug zum drehmomentstarken Anziehen des Verschlussbauteils zur Fixierung eines Stabes, der von der Knochenschraube fixiert werden soll, sowie einem Werkzeug zum Lösen des Verschlusses bekannt.

Das Befestigungswerkzeug umfasst einen zentralen Schaft, der an einem Ende zum Eingriff mit einer Schraube bzw. Mutter mit Innengewinde ausgebildet ist. Im zentralen Schaft des Befestigungswerkzeugs ist eine axiale Bohrung vorgesehen, durch die ein Haltewerkzeug eingeführt werden kann, so dass der zentrale Schaft dieses Werkzeug hülsenförmig umgibt. An dem in der axialen Bohrung geführten Haltewerkzeug ist am Ende ein Außengewinde vorgesehen, das mit dem Verschlusselement in Eingriff gelangt, um dieses zu halten.

Es ist Aufgabe der Erfindung, die Fixiermöglichkeit der Knochenschraube zu verbessern, um zum Einen ausreichend hohe Drehmomente zum dauerhaften Fixieren der Schrauben aufbringen zu können, und um zum anderen ein Übertragen von Abstützmomenten auf den Knochen bzw. den Wirbel beim Verschrauben zu vermindern bzw. zu vermeiden.

Diese Aufgabe wird durch ein Befestigungswerkzeug gemäß Anspruch 1 gelöst.

Durch die gegenläufige Verschraubung können beide Schraubelemente gleichzeitig fixiert werden. Dabei dient das jeweilige Abstützmoment, das beim Festschrauben eines Schraubelements auftritt, gleichzeitig als ein die Verschraubung des anderen Schraubelementes unterstützendes Drehmoment. Dadurch können höhere Drehmomente aufgebracht werden, ohne daß dabei ein gefährliches Drehmoment in den Knochen des Patienten eingeleitet wird.

Mit dem erfindungsgemäßen Werkzeug ist es möglich, ein hohes Drehmoment in zwei entgegengesetzte Richtungen aufzubringen, so daß diese sich nahezu aufheben. Ferner ist es dem Benutzer möglich, auf einfache Weise, das Drehmoment für beide Schraubelemente individuell zu variieren.

Weitere vorteilhafte Ausgestaltungen der Erfindung sind Gegenstand der Unteransprüche.

In der nachfolgenden Beschreibung von bevorzugten Ausführungsformen wird die Erfindung unter Bezugnahme auf die Figuren erläutert. Von den Figuren zeigen:
- Fig. 1:: eine Explosionsdarstellung einer ersten Ausführungsform einer Knochenschraube, teilweise in geschnittener Darstellung;
- Fig. 2:: eine teilweise geschnittene Darstellung der ersten Ausführungsform in zusammengebauten Zustand;
- Fig. 3:: eine alternative Ausführungsform einer Knochenschraube in vergrößerter und teilweise geschnittener Darstellung;
- Fig. 4:: die Seitenansicht einer ersten Ausführungsform eines Befestigungswerkzeugs;
- Fig. 5:: die Seitenansicht des Befestigungswerkzeugs aus Fig. 4 in geschnittener Darstellung;
- Fig. 6:: die Seitenansicht einer zweiten Ausführungsform eines Befestigungswerkzeugs;
- Fig. 7:: die Seitenansicht des Befestigungswerkzeugs aus Fig. 6 in geschnittener Darstellung;
- Fig. 8:: einen Ausschnitt C aus Fig. 7 in vergrößerter Darstellung.

### Erste Ausführungsform

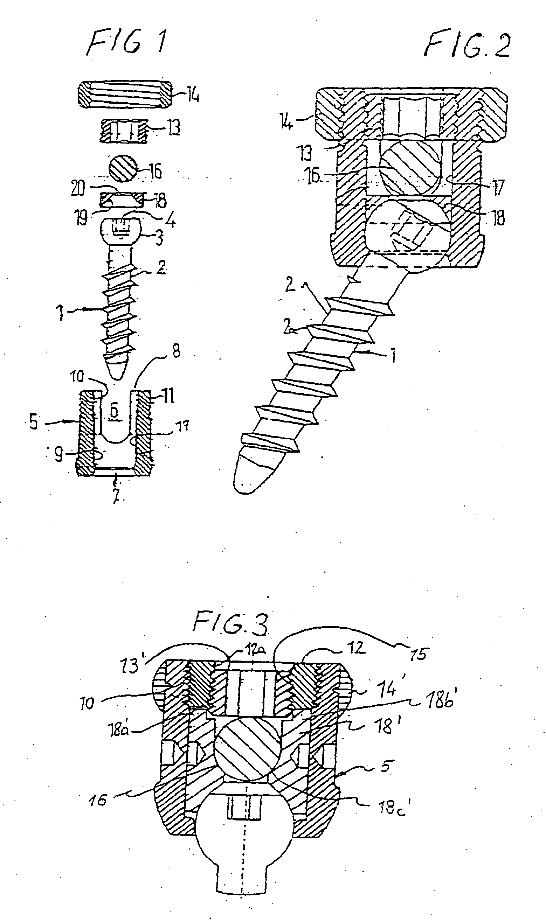

Fig. 2 zeigt eine Knochenschraube in zusammengebauten Zustand. Sie weist ein eigentliches Schraubenelement 1 mit einem Schraubenschaft 2 mit einem Gewindeabschnitt 2a und einem Kopf 3 auf. Der Kopf ist kugelsegmentförmig ausgebildet. Koaxial zur Gewindeachse und auf dem dem Gewindeabschnitt 2a gegenüberliegenden Ende weist der Kopf 3 eine Ausnehmung 4 zum Ineingriffbringen mit einem Inbusschlüssel auf. Die Ausnehmung 4 kann aber auch eine andere geeignete Form haben.

Die Knochenschraube umfaßt ferner ein zylindrisch ausgebildetes Aufnahmeteil 5. Dieses weist an seinem einen Ende eine axialsymmetrisch ausgerichtete erste Bohrung 7 auf, deren Durchmesser größer als der des Gewindeabschnittes 2a und kleiner als der des Kopfes 3 ist. Das Aufnahmeteil 5 weist ferner eine koaxiale zweite Bohrung 8 auf, die auf dem der ersten Bohrung 7 gegenüberliegenden Ende offen ist und deren Durchmesser so groß ist, daß das Schraubenelement durch das offene Ende mit seinem Gewindeabschnitt durch die erste Bohrung 7 hindurch und mit dem Kopf 3 bis zum Grund der zweiten Bohrung 8 führbar ist. Zwischen der ersten und der zweiten Bohrung ist ein kleiner koaxialer Abschnitt 9 vorgesehen, der unmittelbar an die erste Bohrung 7 angrenzt und zum offenen Ende hin sphärisch ausgebildet ist, wobei der Radius im wesentlichen gleich dem Radius des kugelsegmentförmigen Abschnittes des Kopfes 3 ist. Angrenzend an den Abschnitt 9 weist die zweite Bohrung 8 einen Zylinderabschnitt 17 auf, an den sich ein bis zum freien Ende hin erstreckendes Innengewinde 10 anschließt. Das Innengewinde 10 ist in der vorliegenden Ausführungsform als Rechtsgewinde ausgebildet.

Ferner weist das Aufnahmeteil 5 eine Öffnung in Form einer zur Mitte des Teiles symmetrisch angeordneten U-förmigen Ausnehmung 6 auf, deren Grund zu der ersten Bohrung 7 hin gerichtet ist und deren beiden Seitenschenkel sich zu dem der ersten Bohrung 7 abgewandten offenen Ende hin erstrecken. Am freien Ende der Schenkel der U-förmigen Ausnehmung 6 ist ein Außengewinde 11 vorgesehen. Das Außengewinde 11 ist in der vorliegenden Ausführungsform als Linksgewinde ausgebildet.

In dem Zylinderabschnitt 17 wird ein Druckelement 18 aufgenommen, das so ausgebildet ist, daß es auf seiner dem Kopf 3 zugewandten Seite eine sphärische Ansenkung 19 aufweist, deren Radius im wesentlichen gleich dem Radius des kugelsegmentförmigen Abschnittes des Kopfes 3 ist. Der Außendurchmesser des Druckelementes 18 ist so gewählt, daß das Druckelement 18 in dem Zylinderabschnitt 17 eine Gleitbewegung ausführen kann, d.h. in dem Zylinderabschnitt 17 zu dem Kopf 3 hin verschiebbar ist. Vorzugsweise weist das Druckelement 18 eine koaxiale Bohrung 20 auf, die einen Zugriff zur Ausnehmung 4 und auch ein leichteres Zugreifen auf das Druckelement 18 ermöglicht.

Ferner ist eine Fixierschraube 13 vorgesehen, die ein mit dem Innengewinde 10 zusammenpassendes Außengewinde, d.h. ein Rechtsgewinde, aufweist.

Darüber hinaus ist eine Sicherungsmutter 14 vorgesehen, mit einem Innengewinde, das mit dem Außengewinde 11 zusammenpaßt, d.h. mit einem Linksgewinde.

Im Einsatz wird das Schraubenelement 1 vom offenen Ende der zweiten Bohrung 8 her in die erste Bohrung 7 eingeführt. Mit einem entprechenden Werkzeug wie beispielsweise einem Inbusschlüssel kann anschließend das Schraubenelement 2 in den Knochen eingeschraubt werden. Dann werden nacheinander das Drukkelement 18 und der Stab 16 eingesetzt. Das Schraubenelement 1 und das Aufnahmeteil 5 sind in diesem Zustand noch voll gegeneinander verschwenkbar. Danach wird die Fixierschraube 13 in das Innengewinde 10 des Aufnahmeteils 5 eingeschraubt. Dabei drückt die Fixierschraube 13 gegen den Stab 16, der wiederum gegen das Druckelement 18 drückt, das schließlich den Kopf 3 gegen die Bohrung 7 drückt. Dadurch werden das Schraubenelement 1 und das Aufnahmeteil 5 zueinander sowie der Stab 16 fixiert.

Gleichzeitig oder danach wird die Sicherungsmutter 14 in zur Schraubrichtung der Fixierschraube 13 entgegengesetzter Schraubrichtung auf das Außengewinde 11 des Aufnahmeteils 5 aufgeschraubt. Die Sicherungsmutter 14 und die Fixierschraube 13 werden getrennt voneinander solange gedreht, bis jedes der beiden Teile die gewünschte Haltekraft auf den Stab 16 ausübt.

Durch die gegenläufigen Gewinde sind beim Anziehen der Fixierschraube 13 und der Sicherungsmutter 14 die Verschraubungsdrehrichtungen gegenläufig. Der wesentliche Vorteil liegt darin, daß der Operateur beim Anziehen ein stärkeres Drehmoment an beiden Verschraubungen aufbringen kann, ohne daß ein Drehmoment in den Knochen eingeleitet wird, bzw. daß durch jeweiliges Gegenhalten des einen Werkzeugs beim Anziehen der Verschraubung mit dem anderen Werkzeug die Verschraubungen wieder gelöst werden. Vielmehr wird die Festigkeit der Verschraubungen durch das gegenseitige Abstützen der Gegendrehmomente in die Anziehrichtung der jeweiligen Verschraubung gesichert oder gar verstärkt.

### Zweite Ausführungsform

Im folgenden wird eine zweite Ausführungsform gemäß Fig. 3 beschrieben. Übereinstimmende Teile sind jeweils mit den gleichen Bezugszeichen versehen und es wird auf deren Beschreibung in der ersten Ausführungsform verwiesen.

Das Schraubenelement 1 sowie das Aufnahmeteil 5 stimmen mit der ersten Ausführungsform überein. Abweichend von der oben beschriebenen Ausführungsform ist eine Kopf-Fixierschraube 12 und eine Stab-Fixierschraube 13' vorgesehen. Die Kopf-Fixierschraube 12 ist zwischen dem Innendurchmesser 10 des Aufnahmeteils 5 und dem Außendurchmesser der Stab-Fixierschraube 13' vorgesehen. Die Stab-Fixierschraube 13' hat einen kleineren Außendurchmesser als die Fixierschraube 13 der ersten Ausführungsform.

Die Kopf-Fixierschraube 12 hat ein Außengewinde, das mit dem Innengewinde 10 des Aufnahmeteils zusammenpaßt. In der vorliegenden Ausführungsform handelt es sich hierbei um ein Rechtsgewinde. Ferner ist die Kopf-Fixierschraube 12 mit einer koaxialen Bohrung versehen, die ein Innengewinde 15 aufweist, das mit dem Außengewinde der Stab-Fixierschraube 13' zusammenpaßt. In der vorliegenden Ausführungsform handelt es sich hierbei um ein Linksgewinde.

Im Unterschied zur ersten Ausführungsform ist das Druckelement 18' als längliches zylindrisches Element ausgebildet, daß sich vom Kopf 3 bis über den Stab 16 hinaus an die Unterseite der Kopf-Fixierschraube 12 erstreckt. Das Druckelement 18' hat eine zum zweiten Ende 8 hin offene U-förmige Ausnehmung 18c', die sich parallel zur U-förmigen Ausnehmung 6 des Aufnahmeteils 5 erstreckt, zur Aufnahme des Stabes 16. Der Grund ist zylinderförmig ausgebildet, so daß der Stab 16 aufgenommen werden kann. Die in Richtung der Zylinderachse des Aufnahmeteils 5 gesehene Tiefe der U-förmigen Ausnehmung des Druckelements 18' ist größer als der Durchmesser des aufzunehmenden Stabes 16, so daß das Druckelement 18' mit seitlichen Schenkeln 18a' und 18b' über den Stab 16 nach oben vorsteht. Am Grund der U-förmigen Ausnehmung kann sich eine koaxiale Bohrung anschließen, deren Durchmesser kleiner als der Durchmesser des aufzunehmenden Stabes 16 ist.

Im Einsatz wird zunächst wie in der ersten Ausführungsform das durch die zweite Bohrung 8 in die Bohrung 7 eingesetzte Schraubenelement 1 mit dem bereits integrierten Druckelement 18' durch Einschrauben der Kopf-Fixierschraube 12 so auf den Kopf 3 gedrückt, daß dieser eine vorläufige Drehstabilisierung erfährt. Dann wird der Stab 16 in die U-förmigen Ausnehmungen des Aufnahmeteils und des Druckelementes eingelegt. Anschlie-ßend wird die Stab-Fixierschraube 13' in die Kopf-Fixierschraube 12 eingeschraubt und drückt gegen den Stab 16 in den Grund der Ausnehmung des Druckelements 18' und fixiert den Stab 16.

Schließlich können gleichzeitig sowohl die Stab-Fixierschraube 12 im Uhrzeigersinn als auch die Kopf-Fixierschraube 13' im Gegenuhrzeigersinn mit der gewünschten Haltekraft angezogen werden.

Beiden Ausführungsbeispielen ist gemeinsam, daß in den Schraubelementen jeweils Schlitze, Bohrungen, etc. vorgesehen sind, die es erlauben, mit einem Drehwerkzeug gedreht zu werden. Vorteilhafterweise werden die beiden Schrauben gleichzeitig angezogen, da dann die Vorteile der gegenläufigen Drehmomente zum Tragen kommen.

Bei dieser Ausführungsform sind die Verschraubungsdrehrichtungen beim Anziehen der Stab-Fixierschraube 13' und der Kopf-Fixierschraube 12 gegenläufig. Der wesentliche Vorteil liegt darin, daß der Operateur beim Anziehen ein stärkeres Drehmoment an beiden Verschraubungen aufbringen kann, ohne daß ein Drehmoment in den Knochen eingeleitet wird, bzw. daß durch jeweiliges Gegenhalten des einen Werkzeuges beim Anziehen der - Verschraubung mit dem anderen Werkzeug die Verschraubungen wieder gelöst werden. Vielmehr wird die Festigkeit der Verschraubungen durch das gegenseitige Abstützen der Gegendrehmomente in die jeweilige Anziehrichtung der jeweiligen Verschraubung gesichert oder gar verstärkt.

Im übrigen können die jeweiligen Drehrichtungen der Gewinde selbstverständlich auch umgekehrt vorgesehen werden, d.h. was in den obigen Ausführungsformen als Rechtsgewinde ausgeführt war, kann als Linksgewinde ausgeführt sein, und was in den obigen Ausführungsformen als Linksgewinde ausgeführt war, kann als Rechtsgewinde ausgeführt sein.

### Erste Ausführungsform eines Werkzeuges

Im Folgenden wird ein Werkzeug, im nachfolgenden als Befestigungswerkzeug bezeichnet, beschrieben, das dazu geeignet ist, die beiden Verschraubungen der beiden vorstehend beschriebenen Ausführungsformen gleichzeitig anzuziehen.

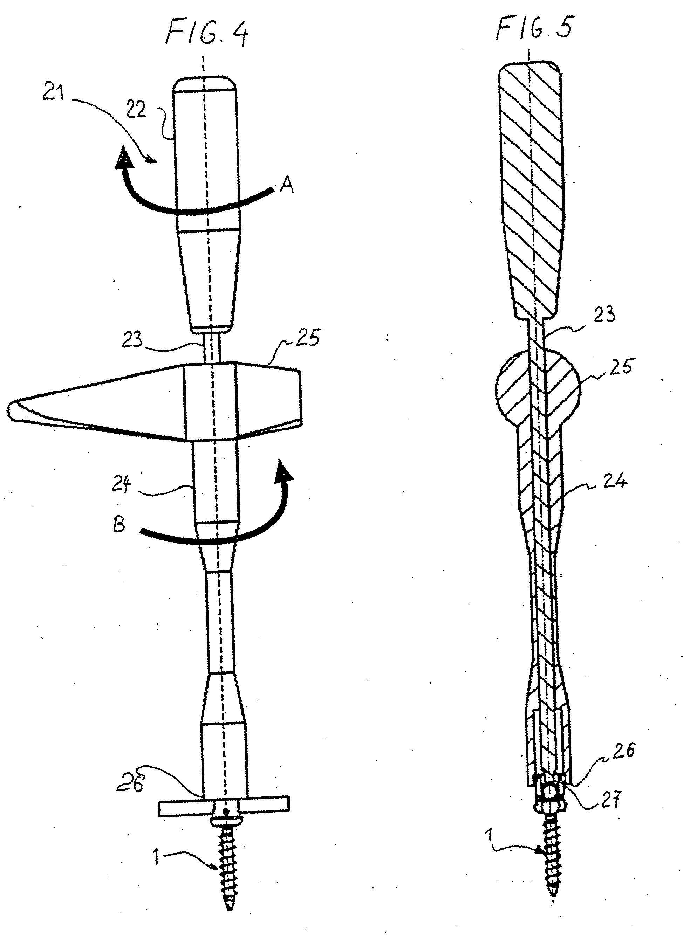

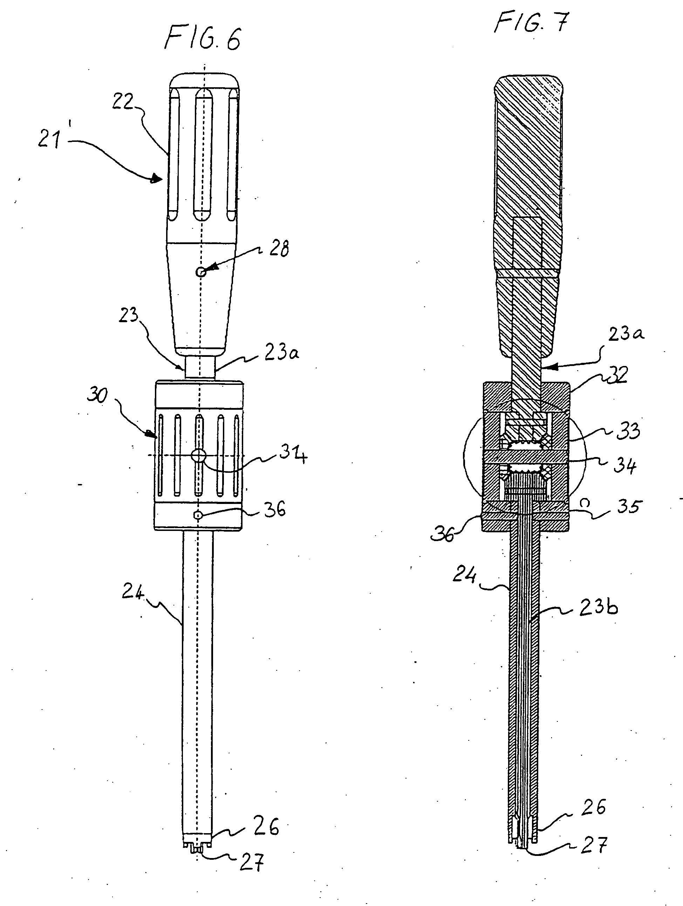

Die Fign. 4 und 5 zeigen eine erste Ausführungsform eines Befestigungswerkzeugs 21. Das Befestigungswerkzeug 21 weist einen ersten Griff 22 auf, von dem sich ein erster Schaft 23 aus erstreckt. Der erste Schaft ist drehfest mit dem ersten Griff 22 verbunden. Am Ende des Schafts 23 ist ein erstes Schraub- bzw. Eingreifelement 27 vorgesehen. Der erste Schaft 23 ist von einem zweiten, hohlen Schaft 24 umgeben. Ein dem ersten Griff 22 zugewandtes Ende des zweiten, hohlen Schaftes 24 ist als zweiter Griff 25 ausgebildet. Am anderen Ende des zweiten, hohlen Schaftes 24 ist ein zweites Schraub- bzw. Eingreifelement 26 vorgesehen. Das zweite Schraub- bzw. Eingreifelement 26 umgibt das erste Schraub- bzw. Eingreifelement 27 konzentrisch. Der erste Schaft 23 und der zweite, hohle Schaft 24 sind gegeneinander verdrehbar (Pfeile A und B in Fig. 4).

### Funktionsweise

Um eine Knochenschraube beispielsweise der zweiten Ausführungsform zu fixieren wird ein erstes und zweites Schraub- bzw. Eingreifelement 26, 27 mit der Kopf-Fixierschraube 12 und der Stab-Fixierschraube 13' in Eingriff gebracht. Eine Hand umgreift dabei den ersten Griff 22, während die andere Hand den zweiten Griff 25 umgreift. Durch Drehen der beiden Griffen in entgegengesetzte Drehrichtungen werden die ersten und zweiten Schraub- bzw. Eingreifelemente 26, 27 ebenfalls in entgegengesetzte Richtungen verdreht und angezogen. Es ist auch möglich, die Drehbewegungen des ersten oder des zweiten Griffs 22, 25 unterschiedlich durchzuführen oder beispielsweise nur einen Griff zu drehen und den anderen Griff lediglich zu fixieren. Das heißt, die Drehbewegungen können je nach Bedarf an Drehmoment variiert werden. Da die Drehmomente in entgegengesetzten Drehrichtungen wirken, wird kein Drehmoment in die Knochenschraube eingeleitet, wodurch eine Gefährdung des Patienten ausgeschlossen werden kann. Vielmehr heben sich die gegenläufigen Drehmomente gegenseitig auf, so daß kein Drehmoment auf das Schraubenelement 1 wirkt. Ein weiterer Vorteil liegt darin, daß durch die konzentrische Anordnung der ersten und zweiten Schraub- bzw. Eingreifelemente 26, 27 nur ein kleiner Raum benötigt wird, was bei einer Operation von großem Vorteil ist, da das Operationsfeld stets nur begrenzt ist.

### Zweite Ausführungsform eines Befestigungswerkzeugs

Im Folgenden wird ein Befestigungswerkzeug gemäß einer zweiten Ausführungsform beschrieben. Übereinstimmende Teile sind jeweils mit den gleichen Bezugszeichen versehen und es wird auf deren Beschreibung in der vorigen Ausführungsform verwiesen.

Die Fign. 6 bis 8 zeigen eine zweite Ausführungsform eines Befestigungswerkzeugs 21'. Wie in der ersten Ausführungsform weist das Befestigungswerkzeug 21' einen ersten Griff 22 auf, von dem sich ein erster, zentraler Schaft 23 aus erstreckt. Der zentrale Schaft ist drehfest mit dem ersten Griff 22 verbunden. Am Ende des zentralen Schaftes 23 ist ein erstes Schraub- bzw. Eingreifelement 27 vorgesehen.

Im Unterschied zur ersten Ausführungsform besteht der erste Schaft aus einem ersten Schaftabschnitt 23a und einem zweiten Schaftabschnitt 23b.

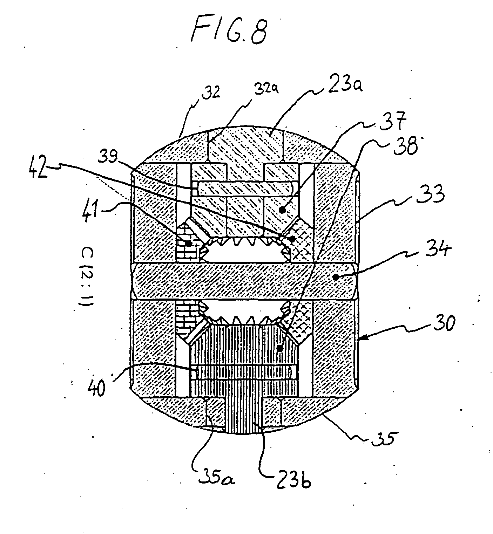

Zwischen dem ersten Schaftabschnitt 23a und dem zweiten Schafttabschnitt 23b ist ein Getriebe angeordnet. Das Getriebe ist in den Fign. 7 und 8 im Schnitt dargestellt. Im folgenden wird auf die vergrößerte Darstellung in Fig. 8 verwiesen. Das Getriebe besteht aus einem Getriebegehäuse 30, das aus einem ersten Deckel 32, einem im wesentlichen zylindrischen Hülsenbauteil 33 und einem zweiten Deckel 35 zusammengesetzt ist. Der erste und der zweite Deckel 32, 35 ist jeweils über nicht dargestellte Senkschrauben mit dem Hülsenbauteil 33 verschraubt. Der erste Deckel 33 besitzt eine zentrale Bohrung 32a, durch die der erste Schaftabschnitt 23a ins Innere des Getriebegehäuses ragt. Gegenüber fluchtend ist im zweiten Deckel 35 ebenfalls eine Bohrung 35a vorgesehen, durch die der zweite Schaftabschnitt 23b ins Innere des Getriebegehäuses ragt. An den im Inneren des Getriebegehäuses 30 befindlichen Enden der ersten und zweiten Schaftabschnitte 23a, 23b sind Zahnräder 37, 38 drehfest angeordnet. Die Befestigung der beiden Zahnräder erfolgt jeweils über Stifte 39, 40, die sich quer zum ersten Schaft durch eine Bohrung erstrecken, die sowohl durch das erste und zweite Zahnrad als auch durch die erste und zweite Schaftabschnitte geht. Dadurch sind die Zahnräder drehfest mit den jeweiligen Schaftabschnitten verbunden. In der vorliegenden Ausführungsform sind die Zahnräder als Kegelräder ausgebildet.

In dem Hülsenbauteil 33 sind quer zur Achsenrichtung des ersten und zweiten Schaftes 23, 24 zwei sich einander gegenüberliegende koaxiale Bohrungen angeordnet, durch die eine Achse 34 gesteckt wird. Auf der Achse 34 sind zwei Zahnräder 41, 42 drehbar gelagert. In dieser Ausführungsform sind die Zahnräder Kegelräder. Die Kegelräder 41, 42 wälzen sowohl mit dem Zahnrad 37 des ersten Schaftabschnittes 23a als auch mit dem Zahnrad 38 des zweiten Schaftabschnittes 23b.

Der erste Schaftabschnitt 23a dient dabei als Eingangswelle und der zweite Schaftabschnitt 23b kann als Ausgangswelle die nen. Ein von dem ersten Schaftabschnitt 23a eingeleitetes Drehmoment wird über die Zahnräder 41, 42 an das Zahnrad 38 des zweiten Schaftabschnittes 23b übertragen. Dabei wird die Drehrichtung umgekehrt, so daß der zweite Schaftabschnitt 23b in einer dem ersten Schaftabschnitt 23a entgegengesetzten Richtung dreht.

Desweiteren ist der zweite, hohle Schaft 24 über mindestens einen Stift 36, im vorliegend Fall über zwei Stifte, drehfest mit dem Deckel 35 des Getriebegehäuses verbunden. Dadurch wird bei Drehen des Getriebegehäuse der zweite, hülsenförmige Schaft 24 ebenfalls gedreht. Durch die Kopplung des hülsenförmigen Schaftes 24 mit dem Getriebegehäuse und durch die Kopplung der beiden Zahnräder 41, 42 über die Achse 34 mit dem Getriebegehäuse kann bei Festlegen des zweiten Schaftabschnittes 23b eine Drehung des ersten Schaftabschnittes 23a über das Getriebegehäuse 30 auf den hülsenförmigen Schaft 24 übertragen werden.

Wie in der ersten Ausführungsform ist am Ende des zweiten Schaftabschnittes 23b des ersten Schaftes 23 ein erstes Schraub- bzw. Eingreifelement 27 vorgesehen. Ebenso ist der erste Schaft 23 von dem hülsenförmigen Schaft 24 umgeben. Das Getriebegehäuse bildet hier an den dem ersten Griff 22 zugewandten Ende des hülsenförmigen Schaftes 24 einen zweiten Griff 25. Am anderen Ende des hülsenförmigen Schaftes 24 ist ein zweites Schraub- bzw. Eingreifelement 26 vorgesehen. Das zweite Schraub- bzw. Eingreifelement 26 umgibt das erste Schraub- bzw. Eingreifelement 27 konzentrisch. Der zentrale Schaft 23 und der hülsenförmige Schaft 24 sind gegeneinander verdrehbar.

### Funktionsweise

Um beispielsweise eine Knochenschraube der zweiten Ausführungsform zu fixieren wird das erste und das zweite Schraub- bzw. Eingreifelement 26, 27 mit der Kopf-Fixierschraube 12 und der Stab-Fixierschraube 13' in Eingriff gebracht. Eine Hand umgreift dabei den ersten Griff 22, während die andere Hand das Getriebegehäuse 30 umgreift, das als zweiter Griff dient. Durch Drehen der beiden Griffe in entgegengesetzte Drehrichtungen werden die ersten und zweiten Schraub- bzw. Eingreifelemente 26, 27 ebenfalls in entgegengesetzte Richtungen verdreht und angezogen. Es ist auch möglich, die Drehbewegungen des ersten oder des zweiten Griffs bzw. Getriebegehäuses 22, 30 unterschiedlich durchzuführen oder beispielsweise, nur einen Griff zu drehen und den anderen Griff bzw. das Getriebegehäuse lediglich zu fixieren. Das heißt, die Drehbewegungen können je nach Bedarf an Drehmoment variiert werden. Da die Drehmomente in entgegengesetzten Drehrichtungen wirken, wird kein Drehmoment in das Schraubenelement 1 der Knochenschraube eingeleitet, wodurch eine Gefährdung des Patienten ausgeschlossen werden kann. Selbst wenn das Getriebegehäuse 30 lediglich fixiert gehalten wird und ein Drehmoment nur über den ersten Griff 22 in das Befestigungswerkzeug eingeleitet wird, wird das Drehmoment über das Getriebegehäuse 30 sowohl auf den zweiten Schaftabschnitt 23b des zentralen Schaftes 23 als auch auf den zweiten hülsenförmigen Schaft 24 übertragen, wobei die Drehmomente unterschiedliche Richtungen besitzen.

Wie in der ersten Ausführungsform liegt ein weiterer Vorteil darin, daß durch die konzentrische Anordnung der ersten und zweiten Schraub- bzw. Eingreifelemente 26, 27 nur ein kleiner Raum benötigt wird, was bei einer Operation von großem Vorteil ist, da das Operationsfeld stets nur begrenzt ist.

Die Knochenschraube ist nicht auf die hier beschriebende Ausführungsform als Polyaxialschraube beschränkt. Vielmehr kann die Knochenschraube auch als Monoaxialschraube ausgebildet sein, wonach der Schraubenkopf 3 und das Aufnahmeteil 5 einstückig ausgebildet sind.

## Patentansprüche

1. Befestigungswerkzeug (21, 21') zum Fixieren einer Knochenschraube mit einem zentralen Schaft (23), der an einem Ende zum Eingriff mit einer Schraube bzw. Mutter ausgebildet ist und an seinem anderen Ende einen ersten Griff (22) aufweist, einem den zentralen Schaft (23) umgebenden hülsenförmigen Schaft (24), der an einem Ende zum Eingriff mit einer Schraube bzw. Mutter ausgebildet ist und an seinem anderen Ende einen zweiten Griff (25) aufweist, wobei beide Schäfte (23, 24) gegeneinander verdrehbar sind.

2. Befestigungswerkzeug (21') gemäß dem vorhergehenden Anspruch, **dadurch gekennzeichnet, daß** der zentrale Schaft (23) in Längsrichtung in einen ersten Schaftabschnitt (23a) und einen zweiten Schaftabschnitt (23b) aufgeteilt ist, zwischen denen ein Getriebe mit einer mit dem ersten Schaftabschnitt (23a) verbundenen Eingangsseite und einer mit dem zweiten Schaftabschnitt (23b) verbundenen Ausgangsseite angeordnet ist, wobei das Getriebe so ausgebildet ist, daß die Drehrichtung eines auf der Eingangsseite eingehenden Drehmomentes an der Ausgangsseite umkehrbar ist, und wobei das Getriebe ein Getriebegehäuse (30) aufweist, mit dem der hülsenformige Schaft (24) drehfest verbunden ist, so daß das in den ersten Schaftabschnitt (23a) eingeleitete Eingangsdrehmoment als ein entgegengesetzt gerichtetes Ausgangsdrehmoment an dem zweiten Schaftabschnitt (23b) des zentralen Schaftes (23) und/oder als ein zum Eingangsdrehmoment gleichgerichtetes Ausgangsdrehmoment an dem hülsenförmigen Schaft (24) ableitbar ist.

3. Befestigungswerkzeug (21') gemäß dem vorhergehenden Anspruch, **dadurch gekennzeichnet, daß** das Getriebegehäuse (30) den zweiten Griff bildet, so daß ein über den ersten Griff (22) oder das Getriebegehäuse (30) eingeleitetes Drehmoment durch Festlegen des ersten Griffs (22) oder des Getriebegehäuses (30) zu gewünschten Anteilen auf den zentralen Schaft (23) und den hülsenförmigen Schaft (24) in jeweils entgegengesetzten Richtungen übertragbar ist.

4. Befestigungswerkzeug (21') gemäß Anspruch 1 oder 2
**dadurch gekennzeichnet, daß**
der erste Schaftabschnitt (23a) des zentralen Schaftes (23) durch eine im Getriebegehäuse (30) vorgesehene erste Bohrung (32a) drehbar gelagert ist und am Ende mit einem ersten Zahnrad (37) drehfest verbunden ist,
der zweite Schaftabschnitt (23b) des zentralen Schaftes (23) durch eine im Getriebegehäuse (30) vorgesehene zweite Bohrung (35a) drehbar gelagert ist und am Ende mit einem zweiten Zahnrad (38) drehfest verbunden ist, und daß im Getriebegehäuse (30) mindestens ein drittes Zahnrad (41, 42) drehbar gelagert ist, das mit dem ersten und zweiten Zahnrad (37, 38) in Eingriff steht.

5. Befestigungswerkzeug (21') gemäß dem vorhergehenden Anspruch, **dadurch gekennzeichnet, daß** das dritte Zahnrad (41, 42) drehbar auf einer senkrecht zur Achse der beiden Schäfte (23, 24) stehenden Achse (34) gelagert ist.

6. Befestigungswerkzeug (21') gemäß einem der beiden vorhergehenden Ansprüche, **dadurch gekennzeichnet, daß** ein viertes Zahnrad (42) vorgesehen ist, das dem dritten Zahnrad (41) gegenüber angeordnet ist.

7. Befestigungswerkzeug (21') gemäß dem vorhergehenden Anspruch, **dadurch gekennzeichnet, daß** sich die Achse (34) von einer Seite des Getriebegehäuses (30) zur anderen Seite erstreckt und die dritten und vierten Zahnräder (41, 42) gemeinsam darauf gelagert sind.

8. Befestigungswerkzeug (21') gemäß einem der Ansprüche 4 bis 7, **dadurch gekennzeichnet, daß** die Zahnräder (37, 38, 41, 42) als Kegelräder ausgebildet sind.

## Claims

1. Fastening tool (21, 21') for fixing a bone screw with a central shank (23) being formed at one end for engagement with a screw or a nut, and having a first handle (22) at its other end, a sleeve-shaped shank (24) surrounding the central shank (23), wherein the sleeve-shaped shank (24) is formed at one end for engagement with a screw or a nut, and comprising at its other end a second handle (25), wherein both shanks (23, 24) can be rotated with respect to one another.

2. Fastening tool (21') according to the previous claim, **characterized in that** the central shank (23) is split in a first shank section (23a) and a second shank section (23b) in a longitudinal direction, between which a gear is arranged having an input side being connected with the first shank section (23a) and an output side being connected with the second shank section (23b) is arranged, wherein the gear is formed such that the rotation direction of a torque introduced at the input side is reversible at the output side, and wherein the gear comprises a gear housing (30) connected with the sleeve-shaped shank (24) in a non-rotational manner such that the input torque introduced into the first shank section (23a) can be derived as an output torque being directed in an opposite direction at the second shank section (23b) of the central shank(23) and/or as an output torque being in the same rotational direction with regard to the input torque at the sleeve-shaped shank (24).

3. Fastening tool (21') according to the previous claim, **characterized in that** the gear housing (30) forms the second handle such that the torque introduced via the first handle (22) or the gear housing (30) is transferable to the central shank (23) and the sleeve-shaped shank (24) in directions opposite to each other, respectively, by means of fixing the first handle (22) or the gear housing (30) in preferred shares.

4. Fastening tool (21') according to claim 1 or 2, **characterized in that** the first shank section (23a) of the central shank (23) is rotatably supported by a first bore (32a) being provided in the gear housing (30), and which first shank section (23a) is connected at the end with a first gear (37) in a non-rotational manner, that the second shank section (23b) of the central shank (23) is rotatably supported by a second bore (35a) being provided in the gear housing (30) and which second shank section (23b) is connected with a second gear (38) in a non-rotational manner, and that at least a third gear (41, 42) is rotatably supported within the gear housing (30) being in engagement with the first and the second gear (37, 38).

5. Fastening tool (21') according to the previous claim, **characterized in that** the third gear (41, 42) is rotatably supported on an axis (34) being vertical with regard to the axis of the two shanks (23, 24).

6. Fastening tool (21') according to one of the two preceding claims, **characterized in that** a fourth gear (42) is provided being arranged opposite to the third gear (41).

7. Fastening tool (21') according to the previous claim, **characterized in that** the axis (34) extends from one side of the gear housing (30) to the other side and that the third and fourth gears (41, 42) are commonly supported thereon.

8. Fastening tool (21') according to one of claims 4 to 7, **characterized in that** the gears (37, 38, 41, 42) are formed as bevel gears.

## Revendications

1. Outil de fixation (21, 21'), pour fixer une vis à os avec une tige centrale (23), réalisée, à une première extrémité, pour venir en prise avec une vis ou un écrou, et présente, à son autre extrémité, une première poignée (22), une tige (24) en forme de douille, entourant la tige centrale (23), tige réalisée, à une extrémité, pour venir en prise avec une vis ou un écrou, et présente à son autre extrémité une deuxième poignée (25), les deux tiges (23, 24) étant susceptibles de tourner l'une par rapport à l'autre.

2. Outil de fixation (21') selon la revendication précédente, **caractérisé en ce que**
la tige centrale (23) est subdivisée en direction longitudinal, en un premier tronçon de tige (23a) et en un deuxième tronçon de tige (23b), entre lesquels est disposée une transmission avec un côté entrée, relié au premier tronçon de tige (23a), et un côté sortie (23b), relié au deuxième tronçon de tige (23b), la transmission étant réalisée de manière que le sens de rotation d'un couple entrant sur le côté entrée puisse être inversé sur le côté sortie, et la transmission présentant un boîtier de transmission (30), relié de façon assujettie en rotation à la tige (24) en forme de douille, de manière que le couple d'entrée, introduit dans le premier tronçon de tige (23a) puisse être dérivé, en tant que couple de sortie, orienté en sens inverse, au deuxième tronçon de tige (23b) de la tige centrale (23) et/ou en tant que couple de sortie, orienté dans le même sens que le couple d'entrée, sur la tige (24) en forme de douille.

3. Outil de fixation (21') selon la revendication précédente, **caractérisé en ce que** le boîtier de transmission (30) forme la deuxième poignée, de manière qu'un couple de rotation, introduit par la première poignée (22) ou le boîtier de transmission (30), soit transmissible, par fixation de la première poignée (22) ou du boîtier de transmission (30), en des proportions souhaitées, à la tige centrale (23) et à la tige (24) en forme de douille, en des sens chaque fois opposés;

4. Outil de fixation (21') selon la revendication 1 ou 2, **caractérisé en ce que** le premier tronçon de tige (23a) de la tige centrale (23) est monté à rotation, à travers un premier perçage (32a) prévu dans le boîtier de transmission (30), et est relié de façon assujettie à rotation, à l'extrémité, à une première roue dentée (27), le deuxième tronçon de tige (23b) de la tige centrale (23) est monté à rotation, à travers un deuxième perçage (35a) prévu dans le boîtier de transmission (30), et est relié de façon assujettie à rotation, à l'extrémité, à une deuxième roue dentée (38), et **en ce que**, dans le boîtier de transmission (30), est monté à rotation au moins une troisième roue dentée (41, 42), s'engrenant avec la première et la deuxième roues dentées (37, 38).

5. Outil de fixation (21') selon la revendication précédente, **caractérisé en ce que** la troisième roue dentée (41, 42) est montée à rotation sur un axe (34), perpendiculaire à l'axe des deux tiges (23, 24).

6. Outil de fixation (21') selon l'une des deux revendications précédentes, **caractérisé en ce qu'**est prévue une quatrième roue dentée (42), disposée à l'opposé de la troisième roue dentée (41).

7. Outil de fixation (21') selon la revendication précédente, **caractérisé en ce que** l'axe (34) s'étend d'un côté du boîtier de transmission (30) à l'autre côté, et les troisième et quatrième roues dentées (41, 42) sont montées en palier conjointement sur celui-ci.

8. Outil de fixation (21') selon l'une des revendications 4 à 7, **caractérisé en ce que** les roues dentées (37, 38, 41, 42) sont réalisées sous forme de roues coniques.
